# EUROPEAN PATENT APPLICATION

(11) **EP 4 574 973 A1**
(43) Date of publication of application: **25.06.2025**
(21) Application number: 24187399.1
(22) Date of filing: 09.07.2024
(51) Int. Cl.: C12N 15/09, C12N 9/90, C07K 14/43, C12N 15/77, C12P 19/24, C12N 15/63

(54) **CONSTITUTIVE PROMOTER FROM CORYNABCATERIUM AND ITS USE IN THE PRODUCTION OF ALLULOSE.**

(30) Priority: 19.12.2023 KR 20230185437
(71) Applicant: Daesang Corporation, Seoul 03130 (KR)
(72) Inventor: CHOI, Eun Seok, 07789 Seoul (KR); KIM, Baek Joong, 07789 Seoul (KR)
(74) Representative: Kehl, Ascherl, Liebhoff & Ettmayr Patentanwälte Partnerschaft mbB

(57) **Abstract**

One embodiment of the present disclosure provides a promoter consisting of a base sequence represented by SEQ ID NO: 6 or a base sequence represented by SEQ ID NO: 27 and regulating the expression of allulose epimerase in a *Corynebacterium* sp. strain. According to the present disclosure, the novel promoter may constitutively and highly express a target protein, particularly an enzyme, in a *Corynebacterium* sp. strain. For example, by using a recombinant *Corynebacterium* sp. strain transformed with an expression vector including the novel promoter according to the present disclosure, it is possible to economically mass-produce allulose epimerase or economically mass-produce allulose from fructose.

## Description

### CROSS REFERENCE TO RELATED APPLICATIONS

This application is based on and claims priority from Korean Patent Application No. 10-2023-0185437, filed on December 19, 2023, with the Korean Intellectual Property Office, the disclosure of which is incorporated herein in its entirety by reference.

### TECHNICAL FIELD

The present disclosure relates to a novel promoter and uses thereof, and more particularly, to a novel promoter capable of constitutively and highly expressing a target protein, a target protein expression system including the same, and a manufacturing method of allulose using the same.

### BACKGROUND

With the development of molecular biology, various mechanisms that regulate gene expression have been found. The gene expression refers to a series of processes that synthesize proteins according to a code input into the gene through transcription and translation that occur within a cell. In particular, the transcription process is an initial step of gene expression, and is initiated when RNA polymerase binds to a promoter sequence located upstream of the gene with the aid of several auxiliary factors, and a transcription factor (TF) is known to bind directly to the promoter sequence as one of these auxiliary factors. In particular, since regulation of gene expression in prokaryotes occurs primarily at the transcription stage, researchers continue to find new transcription factors and promoters.

Industrially, in order to produce foreign proteins such as enzymes, transformants produced by transforming prokaryotes such as E. coli with a pET-type expression vector including a foreign protein gene are used as expression systems. The prokaryotic expression systems transformed with the pET-type expression vector generally have a disadvantage of requiring expensive expression inducers such as isopropyl-β-D-thiogalactopyranoside (IPTG), and requiring precise control of inducer concentration, equipment, expression induction time, etc.

Meanwhile, there has been proposed an expression system using a *Corynebacterium* sp. strain, which is a Generally Recognized As Safe (GRAS) strain, as a host cell to mass-produce psicose epimerase (or allulose epimerase) that has the activity of converting fructose to allulose (or psicose). Regarding a psicose epimerase (or allulose epimerase) expression system based on a *Corynebacterium* sp. strain, in Korean Patent Registration No. 10-1695830, there is disclosed a gene expression cassette including a nucleotide sequence encoding psicose epimerase, and a regulatory sequence that is operably linked to the upstream thereof and regulates expression of the psicose epimerase in the *Corynebacterium* sp. strain, in which the regulatory sequence includes a transcription promoter derived from E. coli selected from a trc promoter, a Tac 1 promoter, and a Tac2 promoter, or a sod promoter, which is a transcription promoter derived from *Corynebacterium glutamicum.* In general, in the promoter, a RNA polymerase binding site is conserved, and there are binding sites for transcription factors that promote or suppress various RNA expressions within a 5'UTR sequence and a 3'UTR sequence based on a core promoter region, and the arrangement of genes around the promoter sequence is important to improve expression efficiency. Since a psicose epimerase expression system based on a *Corynebacterium* sp. strain disclosed in the prior art has a promoter sequence located in a bidirectional promoter region in which an McrA gene and a sod gene are arranged to be expressed in opposite directions, the expression system requires an advanced regulatory mechanism for bidirectional gene expression and is not suitable as a system for constitutive expression of a desired single target protein. In addition, the psicose epimerase expression system based on a *Corynebacterium* sp. strain disclosed in the prior art has a relatively weak expression intensity and is not suitable for mass production of psicose due to structural hindrance of the promoter and complex regulation of promoters.

### SUMMARY

The present disclosure has been made in an effort to provide a novel promoter capable of constitutively and highly expressing a target protein.

The present disclosure has also been made in an effort to provide a target protein expression system, a manufacturing method of allulose, and the like, as various uses of the novel promoter.

The present inventors manufactured promoters by deleting some fragments of a bidirectional promoter site between a McaA gene and a sod gene in a genome sequence of a *Corynebacterium glutamicum* ATCC 13032 strain to exclude bidirectionality, confirmed that a specific promoter thereof may constitutively and highly express allulose epimerase in a *Corynebacterium* sp. strain, and then completed the present disclosure. Further, the present inventors manufactured a recombinant expression vector by operably linking the promoters with the excluded bidirectionality to a polynucleotide encoding allulose epimerase and transformed *Corynebacterium glutamicum* as a Generally Recognized As Safe (GRAS) strain by introducing the recombinant expression vector, and as a result, confirmed that random mutations occurred in some promoters, and a specific promoter variant among the promoter variants obtained through random mutations significantly increased the expression efficiency of allulose epimerase, and then completed the present disclosure.

An exemplary embodiment of the present disclosure provides a promoter consisting of a base sequence represented by SEQ ID NO: 6 or a base sequence represented by SEQ ID NO: 27 and regulating the expression of allulose epimerase in a *Corynebacterium* sp. strain.

Another exemplary embodiment of the present disclosure provides an allulose epimerase expression cassette including a polynucleotide encoding allulose epimerase and a promoter operably linked thereto. The promoter consists of the base sequence represented by SEQ ID NO: 6 or the base sequence represented by SEQ ID NO: 27.

Yet another exemplary embodiment of the present disclosure provides a recombinant expression vector inserted with an allulose epimerase expression cassette.

Yet another exemplary embodiment of the present disclosure provides a recombinant *Corynebacterium* sp. strain in which a *Corynebacterium* host strain is transformed by introduction of an allulose epimerase expression cassette or a recombinant expression vector inserted with the expression cassette.

Yet another exemplary embodiment of the present disclosure provides a manufacturing method of allulose, including adding and reacting a recombinant *Corynebacterium* sp. strain to a fructose-containing solution.

According to the exemplary embodiment of the present disclosure, the novel promoter may constitutively and highly express a target protein, particularly an enzyme, in a *Corynebacterium* sp. strain. For example, by using a recombinant *Corynebacterium* sp. strain transformed with an expression vector including the novel promoter according to the present disclosure, it is possible to economically mass-produce allulose epimerase or economically mass-produce allulose from fructose

The foregoing summary is illustrative only and is not intended to be in any way limiting. In addition to the illustrative aspects, embodiments, and features described above, further aspects, embodiments, and features will become apparent by reference to the drawings and the following detailed description.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented here.

Hereinafter, the present disclosure will be described in detail.

As used in the present invention, the term 'promoter' refers to a minimum nucleic acid sequence which is operably linked to a target nucleotide sequence that is a transcription target and regulates transcription of the target nucleotide sequence. In addition, the promoter may include promoter components sufficient to express a regulatable promoter-dependent gene induced by a cell-type specific or external signal or agent, and these components may be located at the 5' or 3' portion of the gene. The promoter includes both a conservative promoter and an inducible promoter. The promoter sequence may be derived from prokaryotes, eukaryotes or viruses. In prokaryotes, the promoter is usually defined as a binding site immediately near a transcription start site to which RNA polymerase binds.

As used in the present invention, the term 'homology' refers to the identity to a nucleic acid sequence of a wild type or a mutant having the same activity, and the homology may be compared by calculating the homology between two or more sequences as a percentage (%) visually or by using an easily available comparison program. In addition, the 'homology' is used to represent the identity with a nucleic acid sequence of a wild type or a variant having the same activity.

As used in the present invention, the term 'target protein' is a foreign protein and refers to a protein that cannot normally exist in a transformed strain (or host cell) expressing the protein.

As used in the present invention, the term 'polynucleotide' refers to any non-modified or modified polyribonucleotide (RNA) or polydeoxyribonucleotide (DNA). The polynucleotide includes single- or double-stranded DNA, DNA as a mixture of single- and double-stranded regions, single- and double-stranded RNA, RNA as a mixture of single- and double-stranded regions, or hybrid molecules thereof, but is not limited thereto.

As used in the present invention, the term `operably linked' is defined as a state in which a promoter sequence and a nucleotide sequence encoding a target protein are functionally linked to each other, so that the promoter may regulate the expression of the target protein. For example, when the promoter is capable of controlling expression of a coding sequence (i.e., when the coding sequence is under the transcriptional regulation of the promoter), the promoter is operably linked to the coding sequence, or when a ribosome binding site is located to promote translation, the ribosome binding site is operably linked to the coding sequence. The coding sequence may be operably linked to a regulatory sequence in either a sense or antisense direction.

As used in the present invention, the term `recombinant vector' is defined as a recombinant DNA manufactured by cleaving a promoter variant or a target gene using a restriction enzyme and inserting the promoter variant or the target gene into a vector.

As used in the present invention, the term `expression cassette' refers to a regulatory sequence functionally linked to a nucleotide sequence to be expressed, for example, a polynucleotide sequence encoding allulose epimerase. Accordingly, unlike an expression unit, the expression cassette includes not only a nucleotide sequence that regulates transcription and translation, but also a nucleotide sequence expressed as a protein as a result of transcription and translation.

As used in the present invention, the term `expression vector' is defined as a DNA sequence necessary for transcription and translation of cloned DNA in a suitable host, and specifically, refers to a genetic construct including essential regulatory elements operably linked to an insert such that the insert is expressed when present within a cell of a subject. The expression vector may be manufactured and purified using standard recombinant DNA technique. The type of expression vector is not particularly limited as long as the expression vector has a function of expressing a desired gene and producing a desired protein in various host cells of prokaryotic and eukaryotic cells. The expression vector includes at least a promoter, a start codon, a gene encoding a desired protein, and a stop codon terminator. In addition, the expression vector may also appropriately include DNA encoding a signal peptide, an additional expression regulatory sequence, untranslated regions at the 5' and 3' sides of a desired gene, a selection marker region, a replicable unit, or the like. The selection marker region may be a selection marker gene of an antibiotic for selecting a desired vector.

As used in the present invention, the term `recombinant strain' refers to a cell transformed by introducing a polynucleotide encoding at least one target protein or an expression vector having the polynucleotide into a host cell. Methods for manufacturing a transformant by introducing the expression vector into the host cell include transient transfection, microinjection, transduction, cell fusion, calcium phosphate precipitation, liposome mediated transfection, DEAE dextran-mediated transfection, polybrene-mediated transfection, electroporation, electroinjection, chemical treatment methods such as PEG, methods using gene guns, etc., heat shock, and the like, but are not limited thereto. In addition, the host cell of the recombinant strain is not particularly limited in type as long as the promoter present in the expression vector may operate properly, and is preferably a prokaryotic organism.

As used in the present invention, the term 'substrate' refers to any material or compound that is converted or to be converted into another compound by the action of an enzyme. The terms encompass not only single compounds, but also combinations of compounds such as solvents, mixtures and other materials including at least one substrate, and derivatives thereof.

One aspect of the present disclosure relates to a novel promoter capable of constitutively and highly expressing a target protein. The novel promoter according to an embodiment of the present disclosure consists of a base sequence represented by SEQ ID NO: 6 or a base sequence represented by SEQ ID NO: 27. The present inventors named the promoter consisting of the base sequence represented by SEQ ID NO: 6 as `Pds2', and named the promoter consisting of the base sequence represented by SEQ ID NO: 27 as `Pds4' . The promoter Pds2 is a promoter in which some fragments are deleted from a bidirectional promoter site present between the McaA gene and the sod gene in the genomic sequence of the *Corynebacterium glutamicum* strain to exclude bidirectionality and improve the expression efficiency of the target protein. In addition, the promoter Pds4 is a promoter variant in which 6 bp bases located at positions 195 to 200 of the promoter Pds2 are deleted and new 3 bp bases are inserted, which is caused by a mutation in the ribosome-binding site (RBS) spacer. An expression system including the promoter according to one embodiment of the present disclosure may constitutively and highly express a target protein in a *Corynebacterium* sp. strain. In particular, a promoter according to one embodiment of the present disclosure regulates the expression of allulose epimerase in the *Corynebacterium* sp. strain. Therefore, the promoter Pds2 or promoter Pds4 according to one embodiment of the present disclosure may be used as a promoter for constitutive expression in the *Corynebacterium* sp. strain. The novel promoter according to one embodiment of the present disclosure consists of a base sequence represented by SEQ ID NO: 6 or a base sequence represented by SEQ ID NO: 27, but the equivalent range of the promoter is not necessarily limited thereto. For example, the equivalent range of the novel promoter according to one embodiment of the present disclosure includes a sequence having substantial identity to the base sequence represented by SEQ ID NO: 6 or the base sequence represented by SEQ ID NO: 27. The substantial identity means that any other sequence has a sequence homology of 70% or more, 90% or more, or 98% or more with the base sequence represented by SEQ ID NO: 6 or the base sequence represented by SEQ ID NO: 27 by aligning other sequences to maximally correspond to the base sequence represented by SEQ ID NO: 6 or the base sequence represented by SEQ ID NO: 27 and analyzing the sequence thereof. It will be easily appreciated that those skilled in the art may manufacture a polynucleotide having the same or similar activity within the range having the substantial homology by substituting, adding or deleting one or more bases in the base sequence of the novel promoter using a genetic recombination technique and the like known in the art. Comparison of such a homology may be performed by calculating a homology between two or more sequences as a percentage (%) using a commercially available computer program. Accordingly, the equivalent range of the novel promoter according to one embodiment of the present disclosure may include a base sequence having a homology of 70% or more, 80% or more, 90% or more, 95% or more, or 99% or more with the base sequence represented by SEQ ID NO: 6 or the base sequence represented by SEQ ID NO: 27, within a range in which the function of constitutively and highly expressing the target protein is maintained.

One aspect of the present disclosure relates to a target protein expression system including a novel promoter. The novel promoter may be used for the production of an allulose epimerase expression cassette, a recombinant vector, and a recombinant strain.

The allulose epimerase expression cassette according to one embodiment of the present disclosure includes a polynucleotide encoding allulose epimerase and the above-described promoter operably linked thereto. The promoter is preferably located upstream of the polynucleotide encoding the target protein, allulose epimerase. The promoter, which is a component of the allulose epimerase expression cassette, consists of the base sequence represented by SEQ ID NO: 6 or the base sequence represented by SEQ ID NO: 27. The polynucleotide encoding allulose epimerase, which is a component of the allulose epimerase expression cassette, is not particularly limited to the type as long as the polynucleotide is a polynucleotide encoding an enzyme having the activity of converting fructose into allulose. For example, the allulose epimerase may be derived from *Flavonifractorplautii, Clostridium scidens, Treponema primitia, Ensifer adhaerens* or *Ruminococcus torques,* and preferably derived from *Flavonifractor plautii* when considering the conversion activity of fructose into allulose. Specifically, the allulose epimerase may consist of an amino acid sequence represented by SEQ ID NO: 14, an amino acid sequence represented by SEQ ID NO: 16, or an amino acid sequence represented by SEQ ID NO: 18. The allulose epimerase consisting of the amino acid sequence represented by SEQ ID NO: 14 is a wild-type enzyme derived from *Flavonifractor plautii.* In the allulose epimerase consisting of the amino acid sequence represented by SEQ ID NO: 16, tryptophan (Trp) at position 29 in the amino acid sequence represented by SEQ ID NO: 14 is substituted with lysine (Lys), glycine (Gly) at position 216 is substituted with serine (Ser), and methionine (Met) at position 234 is substituted with isoleucine (Ile). In the allulose epimerase consisting of the amino acid sequence represented by SEQ ID NO: 18, tryptophan (Trp) at position 29 in the amino acid sequence represented by SEQ ID NO: 14 is substituted with lysine (Lys), alanine (Ala) at position 77 is substituted with serine (Ser), glycine (Gly) at position 216 is substituted with serine (Ser), and simultaneously, methionine (Met) at position 234 is substituted with isoleucine (Ile). The polynucleotide encoding the allulose epimerase is not particularly limited to the type, and preferably may consist of a base sequence represented by SEQ ID NO: 15, or include a base sequence having 70% or more, 80% or more, 90% or more, 95% or more, or 99% or more of a homology with the base sequence represented by SEQ ID NO: 15. The base sequence represented by SEQ ID NO: 15 is a polynucleotide encoding allulose epimerase consisting of the amino acid sequence represented by SEQ ID NO: 14. In addition, the polynucleotide encoding the allulose epimerase may consist of a base sequence represented by SEQ ID NO: 17 or a base sequence represented by SEQ ID NO: 19. The base sequence represented by SEQ ID NO: 17 is a polynucleotide encoding allulose epimerase consisting of the amino acid sequence represented by SEQ ID NO: 16, and the base sequence represented by SEQ ID NO: 19 is a polynucleotide encoding allulose epimerase consisting of the amino acid sequence represented by SEQ ID NO: 18. In addition, with respect to the allulose epimerase and the polynucleotides encoding the same, the present disclosure includes the contents disclosed in Korean Patent Registration Nos. 10-1919713, 10-2187354, 10-1656063, 10-1695830, 10-2189458, 10-1539097, 10-1539096, 10-1455759, and 10-1318422, Korean Patent Publication No. 10-2023-0073739, etc.

The allulose epimerase expression cassette according to one embodiment of the present disclosure may further include at least one sequence selected from the group consisting of a replication origin, a multi cloning site (MCS) for cloning a target protein gene, a transcription termination sequence, and a selection marker. The selection marker is used to select cells transformed with a vector, and may be used with markers that impart selectable phenotypes such as drug resistance, auxotrophy, resistance to cytotoxic agents, or expression of surface proteins. For example, the selection marker may be an antibiotic resistance gene marker, such as a Kanamycin antibiotic resistance gene or an Ampicillin antibiotic resistance gene. The allulose epimerase expression cassette according to one embodiment of the present disclosure may preferably include a polynucleotide consisting of a base sequence represented by SEQ ID NO: 25 or a polynucleotide consisting of a base sequence represented by SEQ ID NO: 28. The polynucleotide consisting of the base sequence represented by SEQ ID NO: 25 is an expression cassette fragment in which a promoter consisting of the base sequence represented by SEQ ID NO: 6 and an allulose epimerase gene consisting of the base sequence represented by SEQ ID NO: 19 are sequentially linked to each other. In addition, the polynucleotide consisting of the base sequence represented by SEQ ID NO: 28 is an expression cassette fragment in which a promoter consisting of the base sequence represented by SEQ ID NO: 27 and an allulose epimerase gene consisting of the base sequence represented by SEQ ID NO: 19 are sequentially linked to each other.

The recombinant vector according to one embodiment of the present disclosure is a recombinant expression vector inserted with the above-described allulose epimerase expression cassette. The recombinant expression vector preferably has a structure in which a replication origin, a promoter, a polynucleotide encoding allulose epimerase, a transcription terminator, a Kanamycin resistance gene marker, etc. are sequentially linked to each other.

The recombinant strain according to one embodiment of the present disclosure is a recombinant *Corynebacterium* sp. strain, in which a host cell is transformed by introduction of the above-described allulose epimerase expression cassette or the recombinant expression vector inserted with the expression cassette. The host strain used to manufacture the recombinant *Corynebacterium* sp. strain is not particularly limited in type as long as the host strain is a *Corynebacterium* sp. strain, and for example, may be selected from the group consisting of *Corynebacterium glutamicum, Corynebacterium acetoglutamicum, Corynebacterium acetoacidophilum, Corynebacterium thermoaminogenes, Corynebacterium melassecola,* and *Corynebacterium efficiens.*

One aspect of the present disclosure relates to a manufacturing method of allulose using a target protein expression system including a novel promoter.

The manufacturing method of allulose according to one embodiment of the present disclosure includes adding and reacting a recombinant *Corynebacterium* sp. strain to a fructose-containing solution. The fructose acts as a substrate for allulose epimerase, which is the target protein expressed by the recombinant *Corynebacterium* sp. strain. The fructose-containing solution may further include metal ions such as Ca²⁺ and Mn²⁺ to promote the activity of allulose epimerase. In addition, in the manufacturing method of allulose from fructose, the reaction temperature is in the range of 50 to 70°C, preferably 55 to 65°C, and more preferably 60 to 65°C when considering smooth enzyme expression of the recombinant strain, and the stability and maximum activity of the enzyme, and the reaction pH is in the range of 6.5 to 8, preferably 6.5 to 7.5, and more preferably 6.5 to 7. In addition, in the manufacturing method of allulose from fructose, the fructose concentration of the fructose-containing solution is not particularly limited, but preferably 5 to 75% (w/w), and more preferably 10 to 55% (w/w) based on the total weight of the fructose-containing solution when considering productivity and economy.

Hereinafter, the present disclosure will be described in more detail with reference to Examples. However, the following Examples are only for clearly illustrating the technical features of the present disclosure, but do not limit the protection scope of the present disclosure.

### Example 1: Amplification and obtainment of promoter sequences for expression of D-allulose 3-epimerase

To obtain a bidirectional promoter present in a *Corynebacterium* sp. strain, PCR was performed using a genomic DNA of a *Corynebacterium glutamicum* ATCC 13032 strain as a template and a Pctrl-F primer and Pds-R primer set described in Table 1 below. The obtained amplified product was cloned into a pGEM T-easy vector (Promega Co., USA) and the base sequence was analyzed, and as a result, it was confirmed that the base sequence was a polynucleotide fragment consisting of a base sequence represented by SEQ ID NO: 1 with a length of 336 bp. A site corresponding to the promoter in the polynucleotide fragments consisting of the base sequence represented by SEQ ID NO: 1 was named "Pctrl". The promoter Pctrl consisted of a base sequence represented by SEQ ID NO: 2. In addition, in order to obtain a variant promoter of the promoter Pctrl, PCR for amplifying each variant promoter was performed using a genomic DNA of the *Corynebacterium glutamicum* ATCC 13032 strain as a template and a Pds1-F and Pds-R primer set; a Pds2-F and Pds-R primer set; and a Pds3-F and Pds-R primer set described in Table 1 below. The obtained amplified product was cloned into the pGEM T-easy vector (Promega Co., USA) and the base sequence was analyzed. As a result, the amplified product obtained using the Pds1-F primer and Pds-R primer set was a polynucleotide fragment having a length of 282 bp and consisting of a base sequence represented by SEQ ID NO: 3. A site corresponding to the promoter in the polynucleotide fragments consisting of the base sequence represented by SEQ ID NO: 3 was named "Pds1". The promoter Pds 1 consisted of a base sequence represented by SEQ ID NO: 4. In addition, the amplified product obtained using the Pds2-F primer and Pds-R primer set was a polynucleotide fragment having a length of 236 bp and consisting of a base sequence represented by SEQ ID NO: 5. A site corresponding to the promoter in the polynucleotide fragments consisting of the base sequence represented by SEQ ID NO: 5 was named "Pds2". The promoter Pds2 consisted of a base sequence represented by SEQ ID NO: 6. In addition, the amplified product obtained using the Pds3-F primer and Pds-R primer set was a polynucleotide fragment having a length of 236 bp and consisting of a base sequence represented by SEQ ID NO: 7. A site corresponding to the promoter in the polynucleotide fragments consisting of the base sequence represented by SEQ ID NO: 7 was named "Pds3". The promoter Pds3 consisted of a base sequence represented by SEQ ID NO: 8. The promoter Pctrl is a bidirectional promoter including an intergenic region between a McaA gene and a sod gene in the genome sequence of *Corynebacterium glutamicum* ATCC 13032 strain. The promoters Pds1, Pds2, and Pds3 were promoter variants in which some of the promoter Pctrl sequence were deleted based on the intergenic region with a length of 282 bp.

**[Table 1]**

| SEQ ID NO: | Primer name | Primer base sequence (5'→3') |
|---|---|---|
| 9 | Pctr1-F | CAGCTTGCATGCCTGCAGAAGCGCCTCAT CAGCGGT |
| 10 | Pds1-F | CAGCTTGCATGCCTGCAGAACAGGAATG TTCCTTTC |
| 11 | Pds2-F | CAGCTTGCATGCCTGCAGACCCTACTTAG CTGCCAA |
| 12 | Pds3-F | CAGCTTGCATGCCTGCAGTGAAAAATTTC GTTGCAA |
| 13 | Pds-R | CATTCCAATCGGGTTCATGGGTAAAAAA TCCTTTCG |

### Example 2: Amplification and obtainment of polynucleotide sequences encoding D-allulose 3-epimerase

The present applicants disclosed wild-type D-allulose epimerase derived from *Flavonifractor plautii* and a polynucleotide encoding the same in Korean Patent Registration No. 10-14739180. The wild-type D-allulose epimerase consisted of an amino acid sequence represented by SEQ ID NO: 14, and the polynucleotide encoding the same consisted of a base sequence represented by SEQ ID NO: 15.

In addition, the present applicants disclosed a D-allulose epimerase variant W29K/G216S/M234I with improved conversion rate of fructose to allulose and thermal stability and a polynucleotide encoding the same in Korean Patent Publication No. 10-2021-0132405. In the D-allulose epimerase variant W29K/G216S/M234I, tryptophan (Trp) at position 29 in the amino acid sequence of the wild-type D-allulose epimerase derived from *Flavonifractor plautii* was substituted with lysine (Lys), glycine (Gly) at position 216 was substituted with serine (Ser), and simultaneously methionine (Met) at position 234 was substituted with isoleucine (Ile). The D-allulose epimerase variant W29K/G216S/M234I consisted of an amino acid sequence represented by SEQ ID NO: 16, and the polynucleotide encoding the same consisted of a base sequence represented by SEQ ID NO: 17.

In addition, the present applicants disclosed a D-allulose epimerase variant W29K/A77S/G216S/M234I with improved conversion rate of fructose to allulose and thermal stability and a polynucleotide encoding the same in Korean Patent Publication No. 10-2023-0073739. In the D-allulose epimerase variant W29K/A77S/G216S/M234I, tryptophan (Trp) at position 29 in the amino acid sequence of the wild-type D-allulose epimerase derived from *Flavonifractor plautii* was substituted with lysine (Lys), alanine (Als) at position 77 was substituted with serine (Ser), glycine (Gly) at position 216 was substituted with serine (Ser), and simultaneously methionine (Met) at position 234 was substituted with isoleucine (Ile). The D-allulose epimerase variant W29K/A77S/G216S/M234I consisted of an amino acid sequence represented by SEQ ID NO: 18, and the polynucleotide encoding the same consisted of a base sequence represented by SEQ ID NO: 19.

The present inventors named the D-allulose epimerase variant W29K/A77S/G216S/M234I disclosed in Korean Patent Publication No. 10-2023-0073739 as "FpDPE2". Similarly to the contents disclosed in Korean Patent Publication No. 10-2023-0073739, a polynucleotide (SEQ ID NO: 19) fragment of the D-allulose epimerase variant W29K7A77S/G216S/M234I was inserted into the expression vector pET28a (Novagen) to manufacture a recombinant vector pET28a::FpDPE2. Thereafter, PCR was performed using a recombinant vector pET28a::FpDPE2 as a template and an FpDPE2-F primer and FpDPE2-R primer set described in Table 2 below. The obtained amplified product was cloned into a pGEM T-easy vector (Promega Co., USA) and the base sequence was analyzed, and as a result, it was confirmed that the base sequence was a polynucleotide fragment consisting of a base sequence represented by SEQ ID NO: 20 with a length of 921 bp.

**[Table 2]**

| SEQ ID NO: | Primer name | Primer base sequence (5'→3') |
|---|---|---|
| 21 | FpDPE2-F | CGAAAGGATTTTTTACCCATGAACCCGATTG GAATG |
| 22 | FpDPE2-R | TTTCCCACCCGGGGATCCTTACGCGGTCAGC TCCTT |

### Example 3: Manufacture of linkage fragment of promoter and allulose epimerase variant gene

Using a polynucleotide fragment consisting of a base sequence represented by SEQ ID NO: 1 and a polynucleotide fragment consisting of a base sequence represented by SEQ ID NO: 20 as templates, overlap extension PCR was performed using the set of the Pctrl-F primer described in Table 1 and the FpDPE2-R primer described in Table 2, and an expression cassette fragment Pctrl_FpDPE2 was manufactured by linking the promoter Pctrl-F and an allulose epimerase gene FpDPE2. The expression cassette fragment Pctrl_FpDPE2 was cloned into a pGEM T-easy vector (Promega Co., USA) and the base sequence was analyzed, and as a result, it was confirmed that the base sequence was a polynucleotide fragment consisting of a base sequence represented by SEQ ID NO: 23 with a length of 1,221 bp. In addition, using a polynucleotide fragment consisting of a base sequence represented by SEQ ID NO: 3 and a polynucleotide fragment consisting of a base sequence represented by SEQ ID NO: 20 as templates, overlap extension PCR was performed using a set of the Pds1-F primer described in Table 1 and the FpDPE2-R primer described in Table 2, and an expression cassette fragment Pds1_FpDPE2 was manufactured by linking the promoter Pds1 and an allulose epimerase gene FpDPE2. The expression cassette fragment Pds1_FpDPE2 was a fragment including a polynucleotide consisting of a base sequence represented by SEQ ID NO: 24. In addition, using a polynucleotide fragment consisting of a base sequence represented by SEQ ID NO: 5 and a polynucleotide fragment consisting of a base sequence represented by SEQ ID NO: 20 as templates, overlap extension PCR was performed using a set of the Pds2-F primer described in Table 1 and the FpDPE2-R primer described in Table 2, and an expression cassette fragment Pds2_FpDPE2 was manufactured by linking the promoter Pds2 and an allulose epimerase gene FpDPE2. The expression cassette fragment Pds2_FpDPE2 was a fragment including a polynucleotide consisting of a base sequence represented by SEQ ID NO: 25. In addition, using a polynucleotide fragment consisting of a base sequence represented by SEQ ID NO: 7 and a polynucleotide fragment consisting of a base sequence represented by SEQ ID NO: 20 as templates, overlap extension PCR was performed using a set of the Pds3-F primer described in Table 1 and the FpDPE2-R primer described in Table 2, and an expression cassette fragment Pds3_FpDPE2 was manufactured by linking the promoter Pds3 and an allulose epimerase gene FpDPE2. The expression cassette fragment Pds3_FpDPE2 was a fragment including a polynucleotide consisting of a base sequence represented by SEQ ID NO: 26. Specifically, in a reaction solution added with 100 µM of deoxynucleotide triphosphates dATP, dCTP, dGTP, and dTTP, 1 pM of a primer set was added, and 100 ng of a promoter used as a template and the D-allulose epimerase variant DNA fragment were mixed, respectively, and a PCR reaction was performed at 25 to 30 cycles in the presence of 1 unit of a pfu-X DNA polymerase mixture (Bioneer) using a Thermocycler (TP600, TAKARA BIO Inc., JAPAN).

### Example 4: Manufacture of D-allulose epimerase variant expression vector

The expression cassette fragments Pctrl_FpDPE2, Pds1_FpDPE2, Pds2_FpDPE2 and Pds3_FpDPE2 manufactured in Example 3 above were cleaved with restriction enzymes PstI and BamHI, respectively, and then ligated with a commercial plasmid expression vector pVWEx1 having the same restriction enzyme site to manufacture D-allulose epimerase variant expression vectors pPctrl_FpDPE2, pPds1_FpDPE2, pPds2_FpDPE2 and pPds3_FpDPE2. Thereafter, the D-allulose epimerase variant expression vector was transformed into *Escherichia coli* DH5α strain using a heat shock method (see Sambrook and Russell: Molecular Cloning) to obtain colonies with Kanamycin resistance. The obtained colonies were inoculated into an LB liquid medium containing Kanamycin and cultured O/N at 37°C, and then the recombinant plasmid was extracted and as the sequencing result, it was confirmed that the vector sequences of the clones were identical. The extracted recombinant plasmid was transformed into an E. coli JM110 strain using a heat shock method (see Sambrook and Russell: Molecular Cloning.), and colonies with Kanamycin resistance were obtained.

### Example 5: Manufacture of recombinant Corynebacterium sp. strain expressing D-allulose epimerase variant

The colonies of the recombinant E. coli JM110 strain obtained in Example 4 above was inoculated into an LB liquid medium containing Kanamycin and cultured O/N at 37°C, and then a recombinant plasmid was extracted again, and the extracted recombinant plasmid was transformed into a *Corynebacterium glutamicum* strain using the heat shock method (see Sambrook and Russell: Molecular Cloning). Thereafter, the transformed recombinant *Corynebacterium glutamicum* strain was inoculated onto a 2YT solid medium containing Kanamycin and cultured at 30°C for 24 hrs to obtain colonies having Kanamycin resistance. Thereafter, the obtained colonies were inoculated into a 2YT liquid medium containing Kanamycin and cultured at 30°C for 24 hrs, and then a recombinant plasmid was extracted and the base sequence was analyzed. As a result, random mutations were identified in the promoter Pds2 or the allulose epimerase mutant gene sequence in some recombinant plasmids containing the promoter Pds2, respectively. Table 3 below summarizes the random mutations that occurred in some recombinant plasmids containing the promoter Pds2.

**[Table 3]**

| Classification of colonv | Content of promoter Pds2 sequence mutation | Content of FpDPE2 gene sequence mutation |
|---|---|---|
| colony 1 | Matching | Corresponding to 1 bp deletion of nucleotide (A) at position 10/frameshift mutation |
| colony 2 | Corresponding to 6 bp (TTACCC) deletion and 3 bp (CAT) insertion of nucleotides at positions 195 to 200/Ribosome-Binding Site (RBS) spacer mutation | Matching |
| colony 3 | Matching | Corresponding to 1 bp deletion of nucleotide (C) at position 24/frameshift mutation and acquisition of termination codon (TGA), transcription termination after histidine at position 6 |
| colony 4 | Matching | Corresponding to 1 bp deletion of nucleotide (A) at position 1/loss of start codon (ATG) |
| colony 5 | Matching | Corresponding to 1 bp deletion of nucleotide (G) at position 33/frameshift mutation and acquisition of termination codon (TGA), transcription termination after phenylalanine at position 22 |
| colony 6 | Corresponding to 3 bp (CTA) deletion of nucleotides at positions 180 to 182/RBS surrounding sequence mutation | Matching |
| colony 7 | Substitution of nucleotide at position 101 (T→A), substitution of nucleotide at position 104 (G→A), substitution of nucleotide at position 111 (C→T), and 1 bp deletion of nucleotide (G) at position 112 | Matching |
| colony 8 | Matching | Corresponding to 1 bp deletion of nucleotide (T) at position 2/loss of start codon (ATG) |

As shown in Table 3 above, it is expected that the recombinant plasmid expression vectors recovered from colonies 1, 3, 4, 5 and 8 will not be translated to manufacture a desired allulose epimerase variant due to mutations occurring in the allulose epimerase variant gene sequence. On the other hand, in the case of the recombinant plasmid expression vectors recovered from colonies 2, 6, and 7, mutations occurred in the promoter sequence, and the enzyme gene sequences were identical, so that it was determined that there was a possibility of expression of the desired enzyme. The variant promoter in the recombinant plasmid expression vector recovered from colony 2 was named "Pds4", the variant promoter in the recombinant plasmid expression vector recovered from colony 6 was named "Pds4-1", and the variant promoter in the recombinant plasmid expression vector recovered from colony 7 was named "Pds4-2". The promoter Pds4 consisted of a base sequence represented by SEQ ID NO: 27. In addition, the recombinant plasmid expression vector recovered from colony 2 was renamed pPds4_FpDPE2, the recombinant plasmid expression vector recovered from colony 6 was renamed pPds4-1_FpDPE2, and the recombinant plasmid expression vector recovered from colony 7 was renamed pPds4-2_FpDPE2. The recombinant plasmid expression vector, the expression cassette fragment Pds4_FpDPE2 present in pPds4_FpDPE2, was a fragment containing a polynucleotide consisting of the base sequence represented by SEQ ID NO: 28.

### Example 6: Measurement of conversion rate of fructose to allulose by recombinant Corynebacterium sp. strain and comparison of enzyme expression intensities of promoters

The conversion rate of fructose into allulose was proportional to the expression level of D-allulose epimerase in the *Corynebacterium* sp. strain. The induction intensities of enzyme expression of promoters in the recombinant *Corynebacterium* sp. strain were compared with each other by measuring the conversion rate of fructose to allulose in each recombinant *Corynebacterium* sp. strain.

In order to culture the recombinant *Corynebacterium* sp. strain transformed with the recombinant expression vector, 100 ml of a LB medium containing Kanamycin concentration of 50 µg/ml was contained in a 1 L flask, and 1 ml of the recombinant *Corynebacterium* sp. strain manufactured in Example 5 was inoculated therein. Thereafter, the flask was transferred to a shaking incubator, and the recombinant *Corynebacterium* sp. strain was cultured for 14 hrs while maintaining a temperature condition of 30°C and a shaking condition of 140 rpm, and the culture medium was centrifuged to collect the cells. Thereafter, the recovered cells were added at a concentration of 1 mg/mL to a 50 mM PIPES buffer solution (pH 7.0) containing 30% (w/w) fructose and 1 mM manganese sulfate (MnSO4) metal ions, and the reaction was performed at 62°C for a predetermined time, and then the temperature of the reaction product solution was lowered to 4°C to stop the reaction, and centrifugation was performed under conditions of 16,600×g and 4°C to recover the supernatant. Thereafter, the concentrations of allulose and fructose in the supernatant were measured using high-performance liquid chromatography (HPLC), and the conversion rate of fructose to allulose was calculated from the measured results, and the conversion rate was used as an indicator of enzyme activity. Table 4 below summarizes the conversion rate according to a reaction time when converting fructose into allulose using the recombinant *Corynebacterium* sp. strain manufactured in Example of the present disclosure.

**[Table 4]**

| Promoter in recombinant *Corynebacterium* sp. strain | Conversion rate (%) of fructose into allulose according to reaction time | | |
|---|---|---|---|
| | 2 hr | 4 hr | 8 hr |
| Pctrl | 4.31 | 8.54 | 18.40 |
| Pds1 | 3.02 | 5.45 | 9.20 |
| Pds2 | 4.92 | 10.32 | 22.30 |
| Pds3 | 3.37 | 7.21 | 13.20 |
| Pds4 | 5.67 | 12.32 | 25.30 |
| Pds4-1 | 1.21 | 1.81 | 2.23 |
| Pds4-2 | 2.11 | 3.21 | 5.54 |

As shown in Table 4 above, a recombinant *Corynebacterium* sp. strain introduced with the promoter Pds4 and a recombinant *Corynebacterium* sp. strain introduced with the promoter Pds2 showed a higher conversion rate of fructose into allulose than a recombinant *Corynebacterium* sp. strain introduced with the promoter Pctrl, and particularly, showed the highest conversion rate of fructose into allulose in a recombinant *Corynebacterium* sp. strain introduced with the promoter Pds4. From these results, it may be seen that the enzyme expression induction effects of the promoters Pds4 and Pds2 are stronger than those of the promoter Pctrl. In addition, the promoters Pds4 and Pds2 are expected not to cause a load on the recombinant *Corynebacterium* sp. strains due to forms in which a portion of the promoter Pctrl in the *Corynebacterium* sp. strain has been deleted or mutated, and are determined to be suitable as constitutive expression promoters.

As described above, the present disclosure has been described through Examples above, but the scope of the present disclosure is not necessarily limited thereto, and various modifications may be made without departing from the scope and spirit of the present disclosure. Therefore, the scope of the present disclosure is not limited to a specific embodiment disclosed as the best form, and it should be construed as including all embodiments falling within appended claims of the present disclosure.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

## Claims

1. A promoter consisting of a base sequence represented by SEQ ID NO: 6 or a base sequence represented by SEQ ID NO: 27 and regulating the expression of allulose epimerase in a *Corynebacterium* sp. strain.

2. An allulose epimerase expression cassette comprising a polynucleotide encoding allulose epimerase and the promoter of claim 1 operably linked thereto.

3. The allulose epimerase expression cassette of claim 2, wherein the allulose epimerase is derived from *Flavonifractor plautii, Clostridium scidens, Treponema primitia, Ensifer adhaerens* or *Ruminococcus torques.*

4. The allulose epimerase expression cassette of claim 2, wherein the allulose epimerase consists of an amino acid sequence represented by SEQ ID NO: 14, an amino acid sequence represented by SEQ ID NO: 16, or an amino acid sequence represented by SEQ ID NO: 18.

5. The allulose epimerase expression cassette of claim 2, wherein the polynucleotide encoding the allulose epimerase consists of a base sequence represented by SEQ ID NO: 15, a base sequence represented by SEQ ID NO: 17, or a base sequence represented by SEQ ID NO: 19.

6. The allulose epimerase expression cassette of claim 2, comprising: a polynucleotide consisting of a base sequence represented by SEQ ID NO: 25 or a polynucleotide consisting of a base sequence represented by SEQ ID NO: 28.

7. A recombinant expression vector inserted with the expression cassette of any one of claims 2 to 6.

8. A recombinant *Corynebacterium* sp. strain in which a *Corynebacterium* host strain is transformed by introduction of the expression cassette of any one of claims 2 to 6 or a recombinant expression vector inserted with the expression cassette.

9. The recombinant *Corynebacterium* sp. strain of claim 8, wherein the *Corynebacterium* host strain is selected from the group consisting of *Corynebacterium glutamicum, Corynebacterium acetoglutamicum, Corynebacterium acetoacidophilum, Corynebacterium thermoaminogenes*, *Corynebacterium melassecola,* and *Corynebacterium efficiens.*

10. A manufacturing method of allulose, comprising adding and reacting the recombinant *Corynebacterium* sp. strain of claim 9 to a fructose-containing solution.
